# FASCICULE DE BREVET EUROPEEN

(11) **EP 0 645 096 B1**
(45) Date de publication et mention de la délivrance du brevet: **03.12.1997**
(21) Numéro de dépôt: 94402134.4
(22) Date de dépôt: 26.09.1994
(51) Int. Cl.: A23L 1/236, A61K 9/20, C07C 31/26

(54) **Mannitol pulvérulent de friabilité modérée et son procédé de préparation**
Pulverförmiges Mannitol mit verringerter Versprödung und seine Herstellung
Powdered mannitol of reduced friability and its preparation

(30) Priorité: 28.09.1993 FR 9311513
(43) Date de publication de la demande: 29.03.1995
(73) Titulaire: Roquette Frères, F-62136 Lestrem (FR)
(72) Inventeur: Serpelloni, Michel, F-62660 Beuvry Les Bethune (FR); Boonaert, Jean-Philippe, F-62840 Laventie (FR)
(74) Mandataire: Boulinguiez, Didier

(56) Documents cités:
- EP-A- 0 177 428
- EP-A- 0 179 703

## Description

La présente invention a pour objet un mannitol pulvérulent de friabilité modérée présentant en outre une faible densité et une granulométrie particulière.

Elle vise également un procédé de fabrication dudit mannitol.

L'industrie pharmaceutique est consommatrice de tonnages conséquents de saccharose et de lactose. Ceux-ci sont notamment utilisés en tant qu'excipient dans les formes sèches que sont par exemple les gélules, les poudres à dissoudre, les préparations nutritives pulvérulentes et les comprimés. Ces sucres sont également employés sous forme cristallisée dans la préparation industrielle de solutions et de suspensions buvables.

L'industrie alimentaire, de son côté, utilise aussi des quantités importantes de saccharose pour des raisons similaires, soit à l'état cristallisé dans les formes sèches que sont les aliments sucrés à disperser et à diluer comme par exemple les boissons en poudre et les entremets, soit à l'état de soluté comme lors de la préparation de boissons liquides.

Par ailleurs, le saccharose trouve un emploi certain en tant que support dans différentes industries comme celle des additifs destinés en particulier aux domaines alimentaire et pharmaceutique. Ces additifs peuvent être des arômes, des colorants, des édulcorants intenses, des vitamines, des principes actifs ou encore des matières protéiques comme les acides aminés, et les enzymes.

De nos jours, bon nombre de consommateurs, plus soucieux que par le passé de leur alimentation, évitent autant que possible de consommer des sucres. Pour répondre à cette attente, les industriels ont développé des formulations sans sucre dans lesquelles sont employés à titre d'agent sucrant, des édulcorants intenses ou bien des polyols, dont la caloricité réduite et l'innocuité vis-à-vis des dents sont aujourd'hui clairement établies.

En ce qui concerne le domaine auquel on s'intéressera spécifiquement dans la présente invention, à savoir les excipients pharmaceutiques, les édulcorants massiques utilisés dans l'industrie alimentaire, et les supports d'additifs, plusieurs polyols pulvérulents sont déjà d'usage courant. Il s'agit plus précisément du sorbitol, du xylitol et du mannitol.

Le sorbitol présente l'avantage d'être parmi ces trois polyols, le produit le moins cher. Cela explique sa haute fréquence d'emploi. Il s'agit là d'un excellent excipient notamment en compression, en raison de son aptitude particulière à cristalliser sous forme de cristaux en aiguilles directement compressibles.

On lui reproche toutefois d'être, même lorsqu'il est cristallisé dans sa forme la plus stable, plus hygroscopique que le sucre. C'est ainsi que son écoulement devient difficile voire impossible dès lors qu'une reprise en eau est intervenue. Pour éviter ce problème on retient parfois une granulométrie plus grossière mais alors les temps de dissolution de la poudre deviennent en général trop longs. Il n'empêche que même en procédant de la sorte, le caractère hygroscopique élevé du sorbitol comparativement au sucre, rend dans tous les cas l'utilisation de ce polyol rédhibitoire lorsqu'il est associé à des principes actifs ou des ingrédients très sensibles à l'eau.

Le xylitol, quant à lui, n'est, en dehors de la fabrication de comprimés, guère utilisé en tant qu'excipient. Ceci s'explique par son prix élevé mais également par le fait qu'il ait tendance à prendre en masse dans des conditions normales d'humidité, et ceci encore plus facilement que le sorbitol.

Le mannitol, en raison de la faible hygroscopicité de sa forme cristalline, pourrait constituer un excellent excipient. Malheureusement le produit obtenu par cristallisation dans l'eau à partir d'une solution sursaturée, présente toujours une friabilité excessive. La très faible résistance mécanique des particules fait que le produit a tendance à s'effriter et donc à se réduire en poussière lors d'un convoyage, d'un mélange ou encore d'un transport. Les fines particules créées par ces actions mécaniques sont une source de pollution non négligeable dans les ateliers de fabrication mais peuvent aussi être à l'origine d'explosions. De plus, les propriétés d'écoulement, déjà médiocres, du mannitol cristallisé dans l'eau en raison de la structure orthorhombique de ses cristaux, deviennent particulièrement mauvaises lorsque celui-ci renferme ces fines particules. Ceci nuit en particulier au remplissage et à la vidange des trémies et goulottes d'alimentation mais aussi des sacs d'emballage et des sachets unitaires destinés aux patients et aux consommateurs. Le mannitol obtenu par cristallisation dans l'eau présente par ailleurs, en raison de sa structure cristalline très compacte, une mauvaise aptitude à la dissolution. Ceci est le cas même lorsque le produit est finement broyé car alors, les particules se chargent électrostatiquement et forment des agglomérats qui ne se solubilisent que très lentement. Cette faible vitesse de solubilisation, bien que jugée comme un avantage dans certaines applications particulières, est dans les cas auxquels on s'intéresse ici, toujours considérée comme un inconvénient majeur faisant obstacle à son emploi.

D'autres formes pulvérulentes de mannitol ainsi que les moyens d'obtention de celles-ci sont décrits dans la littérature. On connait en particulier les documents suivants :
- le brevet français n° 2 571 045 dont la demanderesse est titulaire qui concerne un mannitol directement compressible obtenu par turbinage à partir d'un produit fondu. Il décrit les caractéristiques de ce produit comparativement à d'autres poudres de mannitol de l'art antérieur obtenues par granulation humide. Il apparait que tous les produits dont il est question dans ce document présentent toujours une granulométrie très grossière et de ce fait possèdent une mauvaise aptitude à la dissolution. Par ailleurs, les produits obtenus selon ces procédés ont une friabilité très faible, comprise entre 45 et 81 % selon le test très sévère utilisé. Comme la demanderesse l'a constaté, il est très difficile de modifier et d'ajuster cette caractéristique en agissant sur les paramètres de fabrication du procédé exposé.
- le brevet français n° 2 571 046 dont la demanderesse est également titulaire et dans lequel est revendiqué un procédé de préparation de mannitol granulaire directement compressible. Le produit obtenu de la sorte est également grossier puisqu'il présente un diamètre moyen de 620 microns. Il est certes possible d'ajuster la granulométrie du produit en réalisant un broyage plus fin et en réalisant ensuite un tamisage mais le rendement de fabrication diminue alors de façon importante si bien que le coût de revient du produit devient exorbitant. Par contre il n'est pas possible d'ajuster la friabilité de la poudre obtenue selon ce procédé. En effet, celui-ci ne permet que de préparer des produits de très faible friabilité toujours inférieure, comme l'a vérifié la demanderesse, à 80 % dans le test particulièrement sévère indiqué.
- le brevet américain n° 3 341 415 qui a trait à une méthode de préparation d'un excipient pharmaceutique contenant au moins 20 % de mannitol et un sucre additionnel choisi parmi le lactose, le saccharose, l'érythritol, le galactose et le sorbitol. Le principe consiste à fondre le mélange binaire composé du mannitol et du sucre retenu, au-dessus de leurs points de fusion respectifs, et ensuite de refroidir le mélange fondu obtenu de façon à le solidifier sous forme de fines gouttelettes dans de l'air froid. On obtient ainsi une poudre ayant un diamètre moyen compris entre 50 et 200 microns. En dehors du fait que le procédé décrit est très délicat à mettre en place à une échelle industrielle, le produit obtenu est hygroscopique, très compact, peu friable et très difficile à solubiliser dans l'eau. Il ne présente donc pas les caractéristiques souhaitées dans le cadre de la présente invention.
- le brevet américain n° 4 293 570 qui décrit un procédé de préparation directe de poudres présentant une granulométrie inférieure à 20 microns. Ce procédé, à l'image de celui décrit ci-avant, consiste à pulvériser sous forme de gouttelettes un sirop édulcorant présentant une matière sèche très élevée, comprise entre 70 et 99,5 % et à refroidir les gouttelettes obtenues dans un courant d'air froid de façon à les solidifier. Il est dit que le procédé peut convenir pour le mannitol, ce qui est peu vraisemblable compte tenu de la solubilité particulièrement faible de ce polyol comparativement aux autres polyols mentionnés. Il n'empêche que si l'on réussissait quand même à mettre en oeuvre le procédé décrit, il ne permettrait pas de préparer des poudres de mannitol exemptes de poussière et ayant de bonnes propriétés d'écoulement compte tenu de la très faible taille des particules alors obtenues.
- la demande de brevet JP 61-85331 revendiquant un procédé de préparation d'excipients de compression directe, consistant à sécher par nébulisation un mélange contenant à la fois du D-mannitol et un hydrolysat d'amidon. Il ressort de ce document qu'avec moins de 5 % d'hydrolysat d'amidon, l'excipient obtenu selon ce procédé, bien que faiblement hygroscopique lorsqu'il est placé à 75 % d'humidité relative et à une température de 40°C, présente toujours une teneur excessivement élevée en particules de taille inférieure à 200 mesh (75 microns). Cette valeur avoisinant 70 % est abaissée lorsque l'hydrolysat d'amidon représente 15 % et 25 % de l'excipient mais alors ce dernier devient malheureusement trop hygroscopique, cariogène, et ne répond plus aux définitions des Pharmacopées en vigueur. En d'autres termes, ce document n'enseigne pas le moyen de préparer un mannitol pulvérulent contenant peu de fines particules et qui de plus est non hygroscopique et non cariogène.
- la demande de brevet JP 61-85330 qui est relative à un procédé de préparation d'excipients caractérisé par le fait qu'il consiste à sécher par nébulisation du D-mannitol sans le mélanger préalablement à un hydrolysat d'amidon contrairement à la demande ci-dessus déposée par la même société. Il apparait que les produits obtenus dans ces conditions contiennent, à la manière des produits témoins, plus de 50 % de particules de taille inférieure à 200 mesh (75 microns), ce qui est préjudiciable à un écoulement correct du produit.
- le brevet japonais JP 80-36646 décrivant un procédé de préparation de poudres granulées, de sucres alcools cristallisés, consistant à sécher par nébulisation une suspension présentant au moins 75 % de matière sèche. Une maturation préalable pendant 3 à 24 heures à haute température est nécessaire afin d'abaisser la viscosité de la suspension. Le procédé n'est appliqué qu'au sorbitol et au xylitol. Il est signalé qu'il peut convenir dans les mêmes conditions à d'autres polyols tels que le mannitol, mais ceci est peu vraisemblable compte-tenu de la faible solubilité de celui-ci. En ajustant les conditions de façon à ce que le procédé décrit devienne applicable au mannitol, il en résulte que le procédé devient particulièrement complexe à mettre en place industriellement mais aussi coûteux du fait des conditions particulières de préparation de la suspension. D'autre part, le produit obtenu contient toujours une teneur très élevée en fines particules comme le produit décrit dans la demande de brevet JP 61-85330.
- le brevet US 3 145 146 décrit un procédé de modification des caractéristiques physiques du mannitol grâce à un séchage par pulvérisation ainsi que le produit obtenu de la sorte. On utilise pour cela un solvant volatil, de préférence de l'alcool éthylique, du chloroforme contenant éventuellement une huile végétale hydrogénée. Ce solvant volatil contient de préférence un liant qui peut être une paraffine, une gomme, un dérivé de cellulose. Ce liant est introduit avant atomisation. On obtient ainsi une poudre dont la granulométrie est comprise entre 5 et 150 microns. La demanderesse a vérifié que la taille des particules est selon ce procédé, tout comme avec les procédés JP 80-36646 et JP 61-85330 décrits plus haut, toujours très faible si bien que le diamètre moyen des particules est compris entre 50 et 75 microns. Ceci revient à dire qu'au moins 50 % des particules de la poudre ont dans tous les cas une taille inférieure à 75 microns, ce qui est loin d'être idéal pour obtenir un bon écoulement et une absence de poussières dans les ateliers.

Fort de ce constat, la demanderesse a relevé qu'il existait un besoin non satisfait depuis de nombreuses années concernant un excipient qui présenterait à la fois les avantages les plus souvent incompatibles d'être non cariogène et non hygroscopique, de posséder de bonnes propriétés d'écoulement et de dissolution, et de ne pas être à l'origine de poussières ou d'explosions dans les ateliers de production ou de conditionnement. Pour obtenir un tel excipient possédant l'ensemble des caractéristiques fonctionnelles énoncées ci-dessus, la demanderesse a constaté que, contre toute attente, il convenait de choisir parmi les polyols un mannitol relativement pur et de modifier ses caractéristiques physiques en employant un procédé approprié de sorte qu'il présente simultanément une friabilité modérée et non excessive, une granulométrie centrée dépourvue de fines particules, et une structure très peu dense.

L'invention se rapporte donc à un mannitol pulvérulent relativement pur caractérisé en ce qu'il présente :
- une friabilité selon un test I comprise entre environ 40 % et environ 80 %
- une densité apparente, pour une coupe granulométrique de 100 à 200 microns, comprise entre environ 300 et environ 525 g/l,
- et moins de 30 % environ de particules de taille inférieure à 75 microns.

L'invention se rapporte également à un procédé de fabrication d'un mannitol pulvérulent possédant les caractéristiques physiques énoncées ci-dessus, caractérisé en ce qu'il comprend une étape d'atomisation d'une solution ou d'une suspension de mannitol puis une étape de granulation par voie humide du mannitol résultant de ladite étape d'atomisation.

On entend par "relativement pur" une richesse en mannitol, calculée par rapport à la quantité de sucres ou de polyols présents, d'au moins 90 %. Par sucres et polyols, on désigne dans la présente invention les mono et disaccharides sous forme hydrogénée ou non.

Il est particulièrement surprenant qu'un produit présentant les caractéristiques physiques énoncés ci-dessus puisse exister. En effet, d'ordinaire, et ceci est le cas de sucres comme par exemple le saccharose, et le dextrose ou même de polyols comme le sorbitol, plus la densité apparente du produit pulvérulent considéré est faible, plus celui-ci devient friable c'est à dire sensible à une altération de sa granulométrie par action mécanique.

A titre d'exemple, le sorbitol commercialisé par la demanderesse sous la marque NEOSORB^{R} 20/60 DC, possédant une densité élevée, présente une friabilité de 54 % dans le test décrit dans le brevet français n° 2 571 045 alors qu'un sorbitol atomisé comme celui commercialisé par exemple par la société Merck sous la marque KARION^{R} Instant, avec une densité nettement plus faible, présente quant à lui, pour une granulométrie voisine et dans les mêmes conditions de mesure, une friabilité plus élevée, voisine de 75 %.

D'ordinaire, une mesure de densité apparente permet donc d'avoir une bonne idée de la friabilité étant donné que friabilité et densité apparente sont toujours inversement corrélées.

De façon surprenante et inattendue, ceci n'est pas le cas pour un mannitol relativement pur. En effet, la friabilité d'un mannitol pulvérulent de faible densité et conforme à l'invention est, contrairement à ce que l'on observe pour les produits cités plus haut, toujours au plus égale et en général inférieure à celle de poudres de mannitol, de structure compacte et très dense, obtenues par cristallisation dans l'eau. Par conséquent, et contrairement à ce que l'on attendait, un mannitol pulvérulent, même très peu dense, peut être utilisé en raison de sa bonne résistance mécanique en tant que support ou excipient. Il posséde d'ailleurs à ce titre de meilleures propriétés qu'un mannitol cristallisé dans l'eau jugé trop friable.

Pour mesurer la caractéristique première du mannitol conforme à l'invention à savoir la friabilité, on procédera selon le test I qui consiste à soumettre les particules à tester à une action mécanique dans un appareil dénommé friabilimètre. On utilise pour cela un appareil de marque ERWEKA TAP fabriqué par la société ERWEKA (6056 HEUSENSTAMM - R.F.A) tournant à une vitesse de rotation uniforme de 25 tours/minutes, et dans lequel on a introduit 5 billes d'acier identiques d'un diamètre de 17 mm et d'un poids de 18,87 g. Pour réaliser ce test I, on introduit dans la chambre de concassage de ce friabilimètre, une quantité de 15 g de produit ayant une granulométrie comprise entre 100 à 200 microns puis on met l'appareil en rotation pendant 15 minutes.

A la fin de l'expérience, on détermine la proportion pondérale représentée par le résidu retenu sur un tamis d'une largeur de maille de 100 microns.

La valeur de la friabilité correspond au pourcentage de poudre non retenu par le tamis précédemment défini.

La friabilité est d'autant plus grande que le pourcentage en poudre non retenu par le susdit tamis est grande.

Il convient de noter que ce test est basé sur le même principe que celui décrit dans le brevet français n° 2 571 045 mais s'applique à une coupe granulométrique plus fine. De ce fait, il est par rapport à celui décrit dans ce brevet nettement moins sévère étant donné que, pour une poudre donnée, une coupe granulométrique grossière est toujours plus friable qu'une coupe granulométrique plus fine.

Le mannitol pulvérulent conforme à l'invention présente dans ce test I, une friabilité modérée et non excessive, c'est à dire comprise entre environ 40 et 80 %. On préférera un produit possédant une friabilité comprise entre 40 et 68 % et mieux encore entre 45 et 65 % dans le test I.

Concernant la seconde caractéristique physique essentielle du mannitol pulvérulent conforme à l'invention, à savoir sa densité apparente, on utilise pour la mesurer un appareil commercialisé par la société HOSOKAWA sous la marque "Powder Tester" en appliquant la méthode recommandée pour mesurer une densité apparente. Dans ces conditions, le mannitol conforme à l'invention présente une densité apparente particulièrement faible c'est à dire comprise entre environ 300 et environ 525 g/l, de préférence 350 à 510 g/l et plus préférentiellement entre 400 et 495 g/l.

Selon la troisième caractéristique physique essentielle, le mannitol pulvérulent conforme à l'invention possède, de façon à présenter les propriétés d'écoulement et de dissolution caractéristiques d'un support ou d'un excipient idéal, non générateur de poussière dans les ateliers, une granulométrie très particulière et centrée. Il contient toujours moins de 30 % environ de particules inférieures à 75 microns. Le mannitol pulvérulent préféré n'en contient que moins de 25 % voire moins de 15 % ; l'idéal étant, bien que cela soit plus difficile à obtenir, un mannitol pulvérulent ne contenant pas plus de 10 % et mieux encore pas plus de 5 % de particules inférieures à 75 microns.

De plus, le mannitol pulvérulent conforme à l'invention est de préférence quasiment dépourvu de particules inférieures à 40 microns, celles-ci étant les plus polluantes et les plus explosives.

Concernant les particules grossières présentes dans le mannitol pulvérulent conforme à l'invention, ce dernier contient de préférence moins d'environ 40 %, plus préférentiellement moins de 30 %, et mieux encore moins de 20 %, de particules de taille supérieure à 250 microns. Idéalement, le produit conforme à l'invention est pour ainsi dire dépourvu de particules de taille supérieure à 315 microns afin que son aptitude à la dissolution dans l'eau soit excellente.

On peut également caractériser ce mannitol pulvérulent conforme à l'invention par son diamètre moyen et son uniformité de distribution granulométrique. Celle-ci, définie comme étant le rapport de la maille pour laquelle 60 % des particules sont passantes à celle pour laquelle 10 % seulement des particules le sont, est généralement comprise entre 1 et 8 environ, de préférence entre 1 et 5 et plus préférentiellement encore entre 1 et 3. Quant au diamètre moyen, il est compris de préférence entre 100 et 200 microns.

Du point de vue de sa composition chimique, le mannitol pulvérulent conforme à l'invention est relativement pur c'est à dire qu'il présente une richesse en mannitol élevée. La demanderesse a vérifié qu'il s'agissait là d'une condition sine qua non à l'obtention d'un support ou d'un excipient stable et peu hygroscopique. Cette richesse en mannitol, calculée par rapport à la quantité de sucres ou de polyols présents, sera d'au moins 90 %, de préférence supérieure à 95 %, et plus préférentiellement supérieure à 98,5 %, l'idéal étant d'approcher autant que possible la valeur de 100 %.

De plus, le mannitol pulvérulent conforme à l'invention contient en général de très faibles quantités d'eau. Cette teneur est de préférence inférieure à 1 % et plus préférentiellement encore inférieure à 0,3 %.

Par ailleurs, le mannitol pulvérulent de la présente invention, peut comprendre des substances autres que des sucres ou des polyols en quantité plus ou moins importante selon la destination qu'il en est fait.

Parmi les substances susceptibles d'entrer dans la composition du mannitol pulvérulent, on peut citer les colorants, les arômes, les parfums, des principes pharmaceutiques ou vétérinaires, les conservateurs, les acides et leurs sels, les édulcorants intenses, les vitamines, les matières grasses, les matières protéiques telles que les acides aminés, les enzymes ou encore les gélatines, les gommes comme les gommes arabique et adragante, les gommes de base de type chewing-gum, les fibres cellulosiques, la cellulose et ses dérivés comme par exemple l'hydropropylméthylcellulose, les pectines, l'inuline et ses dérivés, les amidons et les hydrolysats d'amidons de faible dextrose équivalent, éventuellement hydrogénés, ou bien encore les minéraux.

Au cas où, pour une raison ou une autre, ces substances viendraient à être introduites dans le produit conforme à l'invention, on portera alors un soin très attentif au choix de celles-ci, en retenant de préférence celles qui parmi elles, ne seraient pas susceptibles d'altérer grandement les trois caractéristiques physiques essentielles du mannitol pulvérulent conforme à l'invention définies ci-avant, ou encore d'altérer la propriété intéressante du mannitol d'être à la fois non cariogène et peu hygroscopique.

En ce qui concerne les caractéristiques fonctionnelles du mannitol pulvérulent conforme à l'invention, la demanderesse a évalué son aptitude à l'écoulement en utilisant l'appareil commercialisé par la société HOSOKAWA. Cet appareil permet de mesurer dans des conditions standardisées et reproductibles l'aptitude à l'écoulement d'une poudre et de calculer une note d'écoulement appelée également indice de Carr. Le mannitol conforme à l'invention présente d'ordinaire une note d'écoulement excellente, comprise entre 70 et 90. Cette valeur est de préférence comprise entre 75 et 90 et plus préférentiellement entre 80 et 90. Cette valeur est très voisine de celles de poudres de mannitol de l'art antérieur obtenus par granulation humide ou encore par extrusion de cristaux obtenus par cristallisation dans l'eau. Ceci est d'autant plus remarquable que par rapport à ces produits antérieurs, le mannitol pulvérulent conforme à l'invention présente une granulométrie nettement plus fine.

Par ailleurs, l'aptitude à l'écoulement du produit faisant l'objet de l'invention est d'ordinaire nettement supérieure à celles de poudres de mannitol obtenues par simple cristallisation dans l'eau ou simple atomisation.

Sans vouloir être lié par une quelconque théorie, on peut penser que l'excellente aptitude à l'écoulement du mannitol de l'invention s'explique par la combinaison de plusieurs de ses caractéristiques physico-chimiques à savoir en particulier sa granulométrie centrée, l'absence de charges électrostatiques importantes à la surface des particules le constituant, sa richesse en mannitol, sa faible hygroscopicité et enfin la forme caractéristique des particules le constituant. Concernant ce dernier point, il faut noter en effet que le mannitol pulvérulent conforme à l'invention comprend des particules, de forme variable, toujours dépourvues d'arêtes vives et composées d'une multitude de microparticules agglomérées entre elles. Au microscope, il se différencie aisément d'un mannitol cristallisé dans l'eau constitué de particules, en forme de strates, d'épaisseur sensiblement constante mais de longueur et de largeur variables. Il se différencie également d'un mannitol obtenu par simple atomisation composé de particules essentiellement sphériques ou encore d'un produit extrudé comprenant des particules anguleuses en forme de pavés assez réguliers.

Une seconde propriété fonctionnelle essentielle du mannitol pulvérulent conforme à l'invention, est celle de se dissoudre très rapidement dans l'eau. Pour mesurer cette vitesse de dissolution, on procède selon un test II qui consiste à introduire dans 150 grammes d'eau déminéralisée et dégazée, maintenue à 20°C et soumise à une agitation à 200 t/minute dans un bécher de 250 ml de forme basse, 5 grammes exactement d'une coupe granulométrique de 100 à 200 microns du produit à tester. Le temps de dissolution correspond au temps nécessaire après introduction de la coupe granulométrique pour obtenir une parfaite limpidité visuelle de la suspension ainsi préparée. Dans ces conditions, le mannitol pulvérulent conforme à l'invention possède en général une vitesse de dissolution inférieure à 30 secondes. Le produit préféré se dissout en moins de 25 secondes alors que le produit idéal nécessite un temps inférieur à 20 secondes. Ces temps sont généralement inférieurs à ceux obtenus avec toutes les poudres de mannitol actuellement commercialisées.

Une troisième propriété très intéressante pour l'ensachage et l'utilisation du mannitol pulvérulent conforme à l'invention est celle de produire très peu de poussières bien que sa granulométrie soit plutôt fine comparativement aux produits décrits dans les brevets français FR 2 571 045 et FR 2 571 046. Cette tendance à produire ou non des poussières peut être mesurée aisément en utilisant l'appareil HOSOKAWA déjà décrit plus haut et en mesurant la dispersibilité du produit à tester comme indiqué dans les notices d'utilisation relatives à cet appareil.

Dans ces conditions, le mannitol pulvérulent conforme à l'invention a une dispersibilité comprise en général entre 10 et 30 et de préférence comprise entre 10 et 25, ce qui dénote une très faible tendance à générer des poussières. On peut constater que le produit de l'invention possède sur ce plan d'aussi bonnes caractéristiques que les poudres granulées ou extrudées de mannitol préalablement cristallisé dans l'eau et de granulométrie grossière. Par contre, sa tendance à produire des poussières est nettement moindre que celles des poudres obtenues par simple cristallisation dans l'eau ou par simple atomisation comme celle décrite dans le brevet JP 61.85330.

Par ailleurs, il faut signaler également d'autres avantages non négligeables pour l'utilisateur du mannitol pulvérulent conforme à l'invention. Ces avantages comme ceux décrits plus haut, lui sont également caractéristiques, en ce sens qu'une simple coupe granulométrique des produits de l'art antérieur ne possèdent pas simultanément l'ensemble de ces propriétés. Parmi ces avantages, on peut citer sa très bonne aptitude à être mélangé en poudre à d'autres produits, sa très bonne résistance au démélange ou à une ségrégation particulaire lorsqu'il est mélangé par exemple à une substance de granulométrie plus fine et sa bonne aptitude à être comprimé pour préparer des tablettes à mâcher.

Le mannitol pulvérulent conforme à l'invention est susceptible d'être obtenu en procédant à une étape d'atomisation d'une solution ou d'une suspension relativement pure en mannitol respectivement à la quantité de sucres ou de polyols présents dans la solution ou la suspension, puis à une étape de granulation par voie humide du mannitol résultant de ladite étape d'atomisation. Il est à préciser que, comme l'a constaté la demanderesse, le produit conforme à l'invention ne peut pas être préparé par une simple atomisation comme cela a déjà été réalisé, ni même par la granulation de cristaux de mannitol obtenus par cristallisation dans l'eau ou dans un autre solvant comme l'alcool.

De façon surprenante et inattendue, la demanderesse a constaté que la combinaison d'une atomisation et d'une granulation permettait contrairement, à l'emploi de techniques connues et applicables au mannitol, d'ajuster la friabilité de sorte qu'elle soit élevée mais non excessive, d'ajuster la densité mais aussi de préparer avec un haut rendement un produit conforme à l'invention sur le plan de sa granulométrie.

En effet, les procédés décrits antérieurement ne permettent pas d'obtenir l'ensemble des caractéristiques souhaitées.

Pour procéder à l'atomisation, on emploie de préférence un sirop sous forme d'une suspension ou bien d'une solution, contenant d'une part de l'eau de façon à ce que la matière sèche soit comprise entre 20 et 70 %, de préférence entre 20 et 60 % et d'autre part du mannitol d'une richesse supérieure à 90 %, de préférence supérieure à 95 % et plus préférentiellement supérieure à 98,5 %.

D'ordinaire le sirop, ayant une température comprise entre 20 et 100°C, est alors atomisé en utilisant un atomiseur classique connu de l'homme du métier, et en choisissant généralement une température d'entrée comprise entre 180 et 350°C et un débit tel que les températures de l'air et du produit atomisé soient en sortie comprises toutes deux entre 70 et 130°C.

On notera que le sirop destiné à être atomisé pourra comprendre également des substances autres que des sucres ou des polyols, notamment lorsque celles-ci ne sont pas sujettes à une dégradation thermique.

On notera également que pour augmenter l'efficacité et la productivité de l'atomiseur, on pourra avoir recours avantageusement à un sirop sous forme de suspension ayant une matière sèche de 50 à 70 % et contenant une proportion élevée en mannitol à l'état solide. Cette suspension pourra sans problème être micronisée et/ou chauffée jusqu'à 140°C, compte tenu de la stabilité chimique très élevée du mannitol de façon à ajuster à l'entrée de l'atomiseur la taille et la proportion en particules de mannitol.

La poudre atomisée est ensuite granulée en employant soit de l'eau ou de la vapeur, soit un sirop de mannitol, soit encore, bien que cela ne soit pas préféré, un sirop comprenant un liant tel qu'une polyvinylpyrolidone, une gomme arabique, une hydroxypropylméthylcellulose, une maltodextrine, un amidon prégélifié, une gélatine ou tout autre liant connu par l'homme du métier pour posséder les propriétés requises.

Dans le cas de l'emploi d'eau, la teneur en eau adéquate est en général de l'ordre de 10 à 20 %, mais peut varier avec la granulométrie de la poudre atomisée.

Dans le cas de l'emploi d'un sirop comprenant un liant, ce dernier représente de 0,1 à 15 %, de préférence de 0,5 à 4 %, de la matière sèche du mannitol pulvérulent conforme à l'invention.

On choisit de préférence dans ce cas un liant non susceptible d'altérer la propriété particulièrement intéressante du mannitol de n'être pas cariogène. La façon la plus simple d'y parvenir et qui est préférée, est d'utiliser en tant que liant le même sirop que celui destiné à l'atomisation comme le permet certains appareils industriels, réalisant successivement une atomisation puis une granulation.

Le mannitol pulvérulent conforme à l'invention peut avantageusement être employé, en raison de la qualité de ses propriétés fonctionnelles mentionnées ci-dessus, en tant qu'agent édulcorant, agent de texture, excipient ou support d'additifs, en particulier dans les domaines alimentaire et pharmaceutique.

Il est également possible d'utiliser le mannitol selon l'invention dans bien d'autres industries. Par exemple, il peut servir de produit de base à la préparation de produits chimiques comme par exemple des ignifugeants, des mousses de polyuréthane, des antigels, des tensio-actifs, de produit plastifiant ou de charge comme par exemple dans les plastiques, les peintures, les résines, les caoutchoucs ou les papiers, ou encore de produit visant à supporter ou à mettre en forme par exemple des principes vétérinaires ou phytosanitaires, des enzymes industrielles, des engrais, des oligo-éléments, des pesticides, des actifs permettant la destruction des rongeurs ou de mammifères nuisibles. Bien d'autres applications du mannitol pulvérulent conforme à l'invention sont évidemment envisageables et permises grâce à la mise en oeuvre de ses propriétés fonctionnelles très avantageuses décrites ci-dessus.

L'invention sera encore mieux comprise à l'aide des exemples qui suivent, lesquels font l'état de certains modes de réalisation et de certaines propriétés avantageuses du mannitol pulvérulent selon l'invention.

### EXEMPLE 1

### Préparation d'un mannitol pulvérulent conforme à l'invention.

On prépare une solution aqueuse de mannitol à 40 % de matière en dissolvant à 75°C des cristaux de mannitol obtenus par cristallisation dans l'eau.

Cette solution maintenue à 75°C est atomisée en employant un appareil pilote commercialisé par la société NIRO sous le nom de Minor Mobile.

La température d'entrée est fixée à 280°C et le débit est réglé de façon à ce que la température de sortie soit voisine de 120°C.

La poudre atomisée obtenue est alors granulée en utilisant un sirop identique à celui entrant dans l'atomiseur, puis séchée par un courant d'air chaud et sec. On obtient ainsi un mannitol pulvérulent présentant les caractéristiques suivantes :
- une richesse en mannitol de 98,9 %
- une teneur en eau mesurée par Karl Fisher de 0,1 %
- une friabilité selon le test I décrit ci-avant de 44 %
- une densité apparente de 457 g/l
- le spectre granulométrique suivant :
   - particules de taille inférieure à 75 microns : 3 % environ
   - particules de taille inférieure à 40 microns : traces
   - particules de taille supérieure à 350 microns : traces
   - particules de taille supérieure à 250 microns: 1 % environ
   - particules de taille supérieure à 200 microns : 2 % environ
   - particules de taille supérieure à 100 microns : 86 % environ
- un diamètre moyen : 135 microns environ
- une uniformité de distribution voisine de 1,5
- une note d'écoulement ou indice de Carr de 79
- une densité tassée de 549 g/l
- une vitesse de dissolution selon le test II de 26 secondes
- une dispersibilité selon le test HOSOKAWA de 22 %

Le mannitol pulvérulent conforme à l'invention présente les propriétés d'un bon excipient à savoir présente une friabilité modérée et non excessive, une bonne aptitude à l'écoulement, une vitesse de solubilisation très grande. Cet excipient présente l'avantage de n'être ni cariogène, ni hygroscopique.

Par ailleurs, le mannitol pulvérulent ainsi obtenu, permet d'obtenir aisément des comprimés en utilisant du stéarate de magnésium à un taux voisin de 2 %.

### EXEMPLE 2

### Comparaison de produits conformes à l'invention et de produits de l'art antérieur.

D'autres produits pulvérulents conformes à l'invention sont préparés en appliquant le procédé décrit dans l'exemple 1 mais en modifiant légèrement les températures, les matières sèches d'entrée, et les débits d'atomisation mais aussi les conditions de granulation de façon à obtenir une gamme d'échantillons.

Les produits obtenus présentent les caractéristiques énoncées dans le tableau ci-dessous.

Les produits conformes à l'invention possèdent tous, contrairement aux produits de l'art antérieur, d'excellentes propriétés fonctionnelles, les rendant ainsi aptes à être utilisés sans inconvénient comme excipients et supports d'additifs non cariogènes et non hygroscopiques en particulier dans l'industrie alimentaire et pharmaceutique.

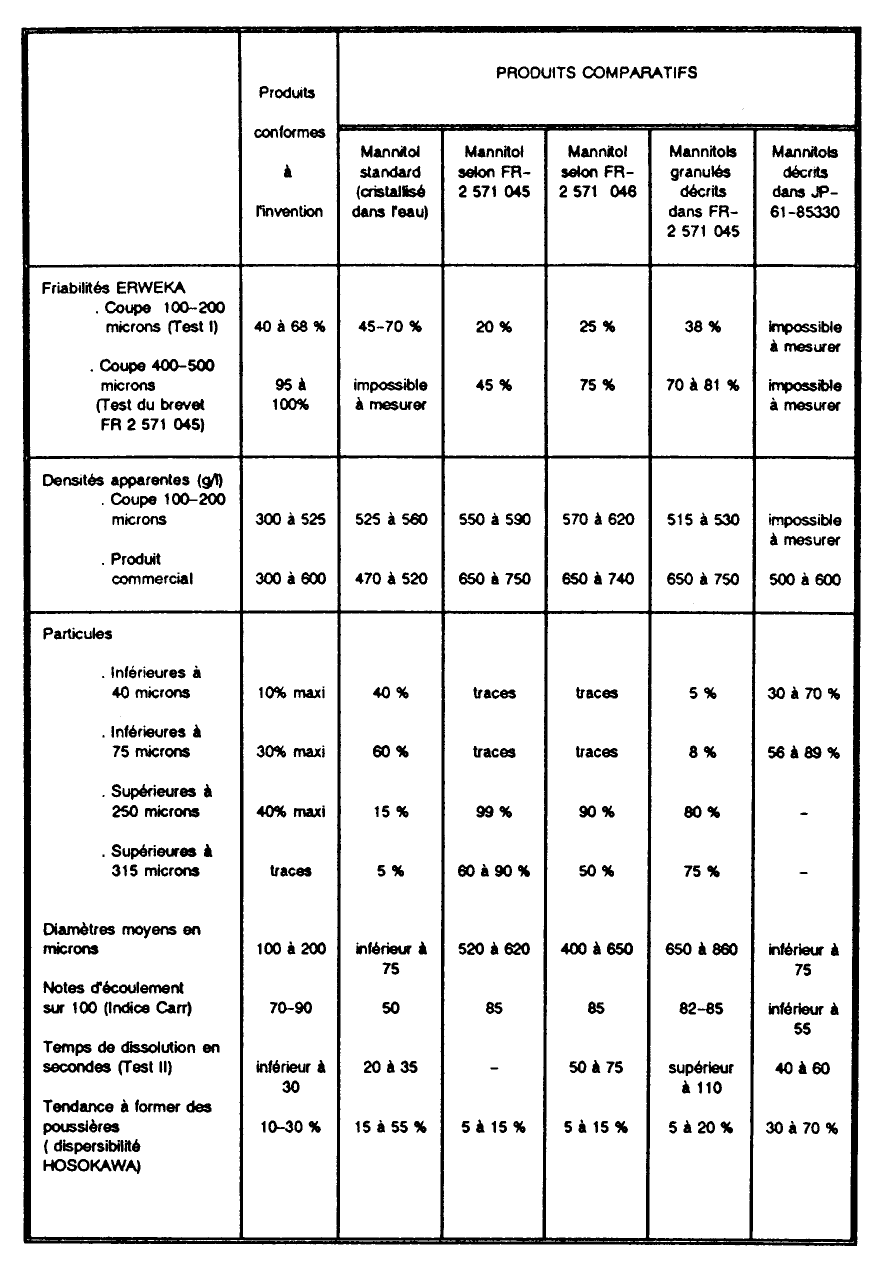

### EXEMPLE 3

### Comparaison de comprimés obtenus par emploi du mannitol pulvérulent selon l'invention à ceux obtenus selon l'art antérieur

On prépare différents comprimés à faces concaves d'épaisseur de 5 mm et de diamètre de 20 mm en employant une presse FETTE P 1000 en exerçant une force de compression de 55 kN.

Pour cela, on utilise les produits pulvérulents suivants, contenant 1 % de stéarate de magnésium :
- **Produit I :**: saccharose granulé ALVEOSUCRE^{R} de Béghin Say (France)
- **Produit II :**: lactose monohydrate sous forme α, TABLETTOSE^{R} de Meggle (Allemagne)
- **Produit III:**: lactose monohydrate sous forme α, atomisé, FAST FLO^{R} de FMC (USA)
- **Produit IV :**: lactose anhydre sous forme α, DC LACTOSE 30 de D.M.V. (Hollande)
- **Produit V :**: mannitol pulvérulent selon l'invention décrit à l'exemple 1.

On mesure la dureté des comprimés obtenus à l'aide d'un duromètre ERWEKA TB24. Les résultats obtenus sont consignés dans le tableau ci-dessous.

| **Produits n°** | **Selon l'art antérieur** | | | | **Invention V** |
|---|---|---|---|---|---|
| | **I** | **II** | **III** | **IV** | |
| **Duretés des comprimés en N** | **50** | **39** | **71** | **52** | **78** |

On constate que le produit conforme à l'invention permet d'obtenir avantageusement des comprimés plus durs qu'avec les différents produits compressibles à base de lactose ou de saccharose couramment utilisés dans cette application.

Le mannitol pulvérulent conforme à l'invention constitue un excellent produit directement compressible contrairement au mannitol cristallisé dans l'eau, et un excellent excipient pour principes actifs les plus divers et notamment ceux sensibles à l'humidité comme par exemple certaines enzymes ou vitamines.

## Revendications

1. Mannitol pulvérulent caractérisé en ce qu'il présente :
- une friabilité selon un test I comprise entre 40 % et 80 %, de préférence entre 40 % et 68 % et plus préférentiellement comprise entre 45 % et 65 %,
- une densité apparente, pour une coupe granulométrique de 100 à 200 microns, comprise entre 300 et environ 525 g/l, de préférence 350 à 510 g/l et plus préférentiellement de 400 à 495 g/l,
- et moins de 30 % environ, de préférence moins de 25 %, et plus préférentiellement moins de 15 % de particules de taille inférieure à 75 microns.

2. Mannitol pulvérulent selon la revendication 1, caractérisé en ce qu'il présente moins de 40 %, de préférence moins de 30 % et plus préférentiellement moins de 20 % de particules de taille supérieure à 250 microns.

3. Mannitol pulvérulent selon la revendication 1, caractérisé en ce qu'il présente une uniformité de distribution granulométrique comprise entre 1 et 8 environ, de préférence comprise entre 1 et 5, et plus préférentiellement comprise entre 1 et 3 et de plus un diamètre moyen compris entre 100 et 200 microns.

4. Mannitol pulvérulent selon la revendication 1 caractérisé en ce qu'il ne présente pas plus de 10 % et de préférence pas plus de 5 % de particules de taille inférieure à 75 microns.

5. Mannitol pulvérulent selon la revendication 1 caractérisé en ce qu'il est quasiment dépourvu de particules de taille inférieure à 40 microns et de particules de taille supérieure à 315 microns.

6. Mannitol pulvérulent selon la revendication 1, caractérisé en ce qu'il présente une richesse en mannitol, calculée par rapport à la quantité de sucres ou de polyols, supérieure à environ 90 %, de préférence supérieure à 95 %, et plus préférentiellement supérieure à 98,5 %.

7. Mannitol pulvérulent selon la revendication 1 caractérisé en ce qu'il présente une note d'écoulement ou indice Carr, comprise entre environ 70 et 90, de préférence comprise entre 75 et 90, et plus préférentiellement entre 80 et 90.

8. Mannitol pulvérulent selon la revendication 1 caractérisé en ce qu'il présente une vitesse de dissolution selon un test II inférieure à 30 secondes, de préférence inférieure à 25 secondes et plus préférentiellement inférieure à 20 secondes.

9. Procédé de préparation du mannitol pulvérulent selon la revendication 1, caractérisé en ce qu'il comprend une étape d'atomisation d'une solution ou d'une suspension de mannitol, puis une étape de granulation par voie humide du mannitol résultant de ladite étape d'atomisation.

10. Compositions destinées en particulier aux domaines alimentaire et pharmaceutique, comprenant le mannitol pulvérulent selon la revendication 1, utilisé en tant qu'agent édulcorant, agent de texture, excipient ou support d'additifs.

## Claims

1. Pulverulent mannitol, characterized in that it presents :
- a friability according to a Test I comprised between 40% and 80%, preferably between 40% and 68% and more preferentially between 45% and 65%,
- an apparent density, for a particle size cut of 100 to 200 microns, comprised between 300 and approximately 525 g/l, preferably 350 to 510 g/l and more preferentially of 400 to 495 g/l,
- and less than approximately 30%, preferably less than 25% and more preferentially less than 15% of particles with a size of less than 75 microns.

2. Pulverulent mannitol according to claim 1, characterized in that it presents less than 40%, preferably less than 30% and more preferentially less than 20% of particles with a size greater than 250 microns.

3. Pulverulent mannitol according to claim 1, characterized in that it presents a uniformity of particle size distribution of between 1 and 8 approximately, preferably of between 1 and 5 and more preferentially of between 1 and 3 and, furthermore, a mean diameter comprised between 100 and 200 microns.

4. Pulverulent mannitol according to claim 1, characterized in that it does not present more than 10% and more preferentially does not present more than 5% of particles with a size of less than 75 microns.

5. Pulverulent mannitol according to claim 1, characterized in that it is virtually free of particles with a size of less than 40 microns and of particles with a size greater than 315 microns.

6. Pulverulent mannitol according to claim 1, characterized in that it presents a mannitol richness, calculated with respect to the amount of sugars or polyols, greater than approximately 90%, preferably greater than 95% and more preferentially greater than 98.5%.

7. Pulverulent mannitol according to claim 1, characterized in that it presents a flow grade or Carr index comprised between approximately 70 and 90, preferably comprised between 75 and 90 and more preferentially between 80 and 90.

8. Pulverulent mannitol according to claim 1, characterized in that it presents a rate of dissolution according to a Test II of less than 30 seconds, preferably of less than 25 seconds and more preferentially of less than 20 seconds.

9. Process for the preparation of the pulverulent mannitol according to claim 1, characterized in that it comprises an atomization stage of a mannitol solution or suspension and then a granulation stage by a wet route of the mannitol resulting from the said atomization stage.

10. Compositions intended in particular for the food and pharmaceutical fields comprising the pulverulent mannitol according to claim 1, used as sweetening agent, texturizing agent or additive excipient or vehicle.

## Patentansprüche

1. Pulverförmiges Mannitol, dadurch gekennzeichnet, daß es aufweist:
- eine Versprödung gemäß einem Test I zwischen 40% und 80%, vorzugsweise zwischen 40% und 68% und ganz besonders bevorzugt zwischen 45% und 65%,
- eine scheinbare Dichte, für einen Korngrößenschnitt von 100 bis 200 Mikrometern, zwischen 300 und etwa 525 g/l, vorzugsweise von 350 bis 510 g/l und ganz besonders bevorzugt von 400 bis 495 g/l,
- und weniger als etwa 30%, vorzugsweise weniger als 25% und ganz besonders bevorzugt weniger als 15% Teilchen mit einer Größe kleiner 75 Mikrometern.

2. Pulverförmiges Mannitol gemäß Anspruch 1, dadurch gekennzeichnet, daß es weniger als 40%, vorzugsweise weniger als 30% und ganz besonders bevorzugt weniger als 20% Teilchen mit einer Größe größer 250 Mikrometern aufweist.

3. Pulverförmiges Mannitol gemäß Anspruch 1, dadurch gekennzeichnet, daß es eine Einheitlichkeit der granulometrischen Verteilung zwischen 1 und etwa 8, vorzugsweise zwischen 1 und 5 und ganz besonders bevorzugt zwischen 1 und 3 und außerdem einen mittleren Durchmesser zwischen 100 und 200 Mikrometern aufweist.

4. Pulverförmiges Mannitol gemäß Anspruch 1, dadurch gekennzeichnet, daß es nicht mehr als 10% und vorzugsweise nicht mehr als 5% Teilchen mit einer Größe kleiner 75 Mikrometern aufweist.

5. Pulverförmiges Mannitol gemaß Anspruch 1, dadurch gekennzeichnet, daß es quasi frei von Teilchen mit einer Größe kleiner 40 Mikrometern und Teilchen mit einer Größe größer 315 Mikrometern ist.

6. Pulverförmiges Mannitol gemaß Anspruch 1, dadurch gekennzeichnet, daß es, berechnet in Bezug auf die Menge an Zuckern oder Polyolen, einen Gehalt an Mannitol größer als etwa 90%, vorzugsweise größer als 95% und ganz besonders bevorzugt größer als 98,5% aufweist.

7. Pulverförmiges Mannitol gemäß Anspruch 1, dadurch gekennzeichnet, daß es eine Fließzahl oder Carr-Zahl zwischen etwa 70 und 90, vorzugsweise zwischen 75 und 90 und ganz besonders bevorzugt zwischen 80 und 90 aufweist.

8. Pulverförmiges Mannitol gemaß Anspruch 1, dadurch gekennzeichnet, daß es eine Auflösungsgeschwindigkeit gemäß einem Test II kleiner 30 Sekunden, vorzugsweise kleiner 25 Sekunden und ganz besonders bevorzugt kleiner 20 Sekunden aufweist.

9. Verfahren zur Herstellung des pulverförmigen Mannitols gemäß Anspruch 1, dadurch gekennzeichnet, daß es einen Schritt der Zerstäubung einer Mannitol-Lösung oder -Suspension, dann einen Schritt der Granulierung auf nasse Weise des aus besagtem Zerstäubungsschritt resultierenden Mannitols umfaßt.

10. Zusammensetzungen, die insbesondere für den Nahrungsmittel- und Arzneimittelbereich bestimmt sind, die das pulverförmige Mannitol gemäß Anspruch 1, verwendet als Süßstoff, Texturmittel, Exzipient oder Träger für Zusatzstoffe, umfassen.
